Europäisches Patentamt

European Patent Office

Office européen des brevets

(11) Publication number: **0 608 873 A1**

## EUROPEAN PATENT APPLICATION

(21) Application number: **94101148.8**

(22) Date of filing: **26.01.94**

(51) Int. Cl.⁵: **C07C 291/04**, C09K 15/32

A request for correction of the description has been filed pursuant to Rule 88 EPC. A decision on the request will be taken during the proceedings before the Examining Division (Guidelines for Examination in the EPO, A-V, 2.2).

(30) Priority: **29.01.93 GB 9301836**

(43) Date of publication of application:
**03.08.94 Bulletin 94/31**

(84) Designated Contracting States:
**AT BE CH DE DK ES FR GB GR IE IT LI LU MC NL PT SE**

�'], Applicant: **ALBRIGHT & WILSON LIMITED**
**P.O. Box 3,**
**210-222 Hagley Road West**
**Oldbury, Warley, West Midlands B68 0NN(GB)**

(72) Inventor: **Ghadimi, Moharam**
**17, Ferndale,**
**Eaglestone**
**Milton Keynes, Buckinghamshire MK7 5AE(GB)**
Inventor: **Sargent, Malcolm Thomas**
**8 Bronte Drive**
**Kidderminster, Worcestershire DY10 3YU(GB)**

(74) Representative: **Savidge, Roger Gordon**
**Madgwick et al**
**Albright & Wilson Limited,**
**Patents Department,**
**P.O. Box 3,**
**210-222 Hagley Road West,**
**Oldbury**
**Warley, West Midlands B68 0NN (GB)**

(54) **Nitrosamine inhibition.**

㊿ A synergistic nitrosamine and/or nitrite inhibitor comprising :-
(i) a compound of formula:-

$$R_yN(O)_x(CH_2PO_3M_2)_{(3-y)}$$

where y is 1 or 2, x is 1 or 0, R is an alkyl group having up to 6 carbon atoms or a hydroxyalkyl, carboxyalkyl or polyoxethylene group having 2 to 6 carbon atoms and M is hydrogen, or a cation such that the compound is water soluble; and
(ii) a synergistic proportion of a compound of general formula :-

$$(M_2O_3PCH_2)_2N(O)_x[(CH_2)_mN(O)_xCH_2PO_3M_2]_nCH_2PO_3M_2$$

wherein n is independently from 0 to 8, m is 2 or 3, and x and M are as hereinbefore defined.

The present invention relates to a novel synergistic nitrosamine and nitrite inhibitor; to a method of inhibiting the formation of nitrosamines and nitrites, especially during the preparation, storage and/or heating of nitrosamine precursors such as amine oxides and alkanolamides; an improved process for the preparation of amine oxides; and compositions containing a nitrosamine precursor and an inhibitor of nitrosamines and nitrites.

## Technical Background

It has been recognised for some years that nitrosamines, which are widely regarded as potentially harmful, and nitrites which may be precursors of nitrosamines, are commonly present as minor but highly undesirable trace contaminants of many amines and their derivatives. For example both contaminants are typically present in commercial amine oxides at low levels of between 200 and 1000 parts per billion (ppb).

Until recently, such levels have been at, or below, the limits of reliable detection. However, improved analytical methods, for instance based on chemiluminescence, have now made it possible to detect total nitrosamine (referred to herein as "total NO") and nitrite down to levels as low as 10ppb or even lower. This is frequently lower than ambient atmospheric levels.

There is now a pressing demand for nitrosamine precursor products containing less than 50ppb total NO. Indeed, even lower levels of total NO in such products would probably be demanded if they were thought to be achievable.

A problem arises with potential nitrosamine precursors such as amine oxides because, in the absence of inhibitors, even products which can be prepared with a low initial concentration of total NO, exhibit rapid and significant increases in total NO levels on standing.

It is possible to prepare nitrosamine precursors with low initial total NO levels , for example by treating said precursor with U.V. radiation to degrade the nitrosamine and nitrite contaminents already present. Although this approach provides a product with a low initial level of contaminants, the U.V. treatment does not protect the product from further degradation to nitrosamines and nitrites during storage.

Theoretically in the preparation of amine oxides it is possible to obtain very low levels of total NO by careful control of the ratio of hydrogen peroxide to amine during the preparation, and ensuring only highly pure reagents are used. This excludes as far as practicably possible the presence of dissolved polyvalent metal ions which favour or accelerate the decomposition of the hydrogen peroxide, which may result in an undesirably high level of free amine in the product. However, in reality the conditions are so sensitive that it is not possible in normal industrial practice to obtain consistently low levels of total NO by this approach. In order to complete the reaction within a commercially acceptable time it is necessary to heat the reaction mixture and/or to employ a catalyst. Since excessive heating tends to cause (or accelerate) the decomposition of peroxide, and the formation of nitrosamine and nitrite, the use of a catalyst and more moderate temperatures is preferred. However, the amine oxide products so produced are not stabilised against the accumulation of NO on storage.

It is known that high levels of bicarbonate can inhibit the formation of nitrosamines during the preparation and storage of amine oxides, especially in the presence of certain phosphonates. The degree of inhibition hitherto achieved, however has not been able, reliably to match the most stringent demands. Moreover high levels of inorganic material such as bicarbonate may be unacceptable to some customers.

## The Problem

The problem is therefore to provide an inhibitor that can give better inhibition of nitrosamine, preferably without requiring very high levels of inorganic salts.

## Prior Art

GB 2 252 320 describes the use of relatively high concentrations of carbonates and/or bicarbonates to inhibit the formation of nitrosamines during the oxidation of amines and to stabilise the amine oxide product against such contamination upon storage.

EP 0 409 043 describes the use of certain aminophosphonates as catalysts in the preparation of amine oxides. British patent application No. 9301761.4 describes a synergism between aminophosphonic acids and bicarbonates and/or relatively low levels of carbonates which permit very low levels of total NO, to be achieved and maintained.

## The Invention

We have discovered that certain alkylamine and alkanolamine (methylene phosphonic) acids, their N-oxides, homologues, water soluble salts or precursors are effective in inhibiting the formation of nitrosamines during e.g. the preparation of amine oxides. In particular we have discovered that such compounds, in conjunction with polyalkyleneamine polymethylene phosphonic acids and their N-oxides, homologues, water soluble salts or precursors, act as synergistic inhibitors of nitrosamines and nitrites during storage of potential nitrosamine precursors.

## Statement of the Invention

The invention provides a synergistic nitrosamine and/or nitrite inhibitor comprising :-
(i) a compound of formula:-

$$R_yN(O)_x(CH_2PO_3M_2)_{(3-y)}$$

where y is 1 or 2, x is 1 or 0, R is an alkyl group having up to 6 carbon atoms or a hydroxyalkyl, carboxyalkyl or polyoxethylene group having 2 to 6 carbon atoms and M is hydrogen, or a cation such that the compound is water soluble; and
(ii) a synergistic proportion of a compound of general formula :-

$$(M_2O_3PCH_2)_2N(O)_x[(CH_2)_mN(O)_xCH_2PO_3M_2]_nCH_2PO_3M_2$$

wherein n is independently from 0 to 8, m is 2 or 3, and x and M are as hereinbefore defined.

In a second embodiment, the present invention provides a method of inhibiting the conversion of potential nitrosamine precursors to nitrosamine and/or nitrite during the preparation, storage and/or heating of said precursors which comprises adding thereto an effective proportion of said synergistic inhibitor.

In a third embodiment, the present invention provides, a synergistic inhibitor comprising (i) an alkanolamine (methylene phosphonic) acid, its N-oxide, or its water soluble salts, and (ii) a polyalkyleneamine poly(methylene phosphonic) acid, its N-oxide, or its water soluble salts.

In a fourth embodiment, the present invention provides, an improved method for the preparation of amine oxides, wherein said method comprises reacting an amine, especially a tertiary amine, with hydrogen peroxide in the presence of a sufficient amount of an inhibitor comprising a hydroxyethylamine bis (methylene phosphonic) acid or its N-oxide or salts.

We prefer that the nitrosamine inhibitor additionally comprises an alkali bicarbonate and/or carbonate.

## Nitrosamine Precursors

The 'nitrosamine precursors' referred to herein, include any amine containing compounds which are susceptible to contamination by nitrosamines and nitrites, and include secondary and tertiary amines and their derivatives, such as amides (e.g. alkanolamides), tertiary amine oxides and quaternary amine compounds (e.g. fatty amine quaternaries, betaines.) Alternatively the amines may comprise cyclic amines such as imidazolines or pyridines, N-substituted piperazines, or N-substituted morpholines e.g. N-methylmorphaline.

In a particularly preferred embodiment of the present invention, the nitrosamine precursor is an amine oxide.

The synergistic nitrosamine and nitrite inhibitor may be present during the preparation of said nitrosamine precursors to produce products with a low initial level of total NO, or alternatively said synergistic inhibitor may be added after preparation of said precursors to maintain low levels of total NO on storage.

## The Synergistic Inhibitor

Compound (i) of the synergistic inhibitor is an alkyl, hydroxyalkyl or carboxyalkylamine (methylene phosphonate) of general formula (1):-

(1)     $R_yN(O)_x(CH_2PO_3M_2)_{(3-y)}$

where y is 1 or 2, x is 1 or 0, R is an alkyl group having up to 6 carbon atoms or a hydroxyalkyl, carboxyalkyl or polyoxyethylene group having 2 to 6 carbon atoms and M is hydrogen, or a cation such that the compound is water soluble. Preferably the compound is ethanolamine bis (methylene phosphonate) or its N-oxide i.e. a compound wherein R is a hydroxyethyl group, M is H or an alkali metal such as sodium or potassium, especially sodium, and n is 1. Preferably (i) is added to the nitrosamine precursor as hydroxyethylamine bis (methylene phosphonic) acid or its salts.

Component (i) is typically added to the reaction mixture as the tertiary amine, for example hydroxyethylamine bis (methylene phosphonic) acid, however we believe that such tertiary amines are rapidly converted to the corresponding amine oxides if oxidising compounds are present (e.g. in the presence of hydrogen peroxide in the reaction to produce amine oxides).

Component (ii) of the synergistic inhibitor is a polyalkyleneamine polymethylene phosphonate of general formula (2) :-

(2)  $(M_2O_3PCH_2)_2N(O)_x[(CH_2)_mN(O)_xCH_2PO_3M_2]_nCH_2PO_3M_2$

Wherein n is independently from 0 to 8, m is 2 or 3 and M is as hereinbefore defined with respect to component (i), wherein inhibitor 2 may also be an N-oxide, acid, homologue or precursor of general formula (2).

It is especially preferred that component (ii) is a polyalkyleneamino poly(methylene phosphonic) acid wherein the value of 'n' is from 2 to 7. In an especially preferred embodiment of the present invention 'n' is from 2 to 5 e.g. a diethylenetriamine pentakis (methylene phosphonate), or the N-oxide thereof.

For both components it is believed that if oxidising conditions exist when the inhibitors are present in the nitrosamine precursor product e.g. during preparation of the nitrosamine precursors, the inhibitors will have x = 1 in the product, even if the tertiary amine form was originally added, since the oxide will be formed in situ. If the stabiliser is post added to the product x may be 0 or 1. Usually it is preferable that at least part of the stabiliser is added to the product after preparation, so as to be present in the composition as the unoxidised tertiary amine. If the components of the synergistic inhibitor are added at different stages i.e. component (i) during the preparation and component (ii) upon completion of the preparation of a nitrosamine precursor product, the synergistic inhibitor may then comprise a component in the oxide form and a component in the tertiary amine form.

It is an especially preferred embodiment of the present invention that the synergistic inhibitor mixture comprises (i) an alkali metal hydroxyethylamine bis (methylene phosphonate) or its N-oxide, and (ii) an alkali metal diethylenetriamine pentakis (methylene phosphonate).

The weight ratio of (i) : (ii) in the synergistic inhibitor lies within the range 15:1 to 1:15, preferably 10:1 to 1:10, more preferably 7:1 to 1:7, especially 5:1 to 1:5.

It is an especially preferred feature of the present invention that the synergistic inhibitor comprises predominance of (i), or equal quantities by weight of (i) and (ii).

The synergistic inhibitor may be added to the nitrosamine precursors either as the mixture, or by separate addition of the two components. The complete synergistic inhibitor may be added to a nitrosamine precursor containing low levels of total NO in order to maintain those low levels of total NO upon storage. For example said products may have received U.V. treatment to reduce the levels of NO, or may have been prepared by methods which inherently give low NO products. Alternatively the complete synergistic inhibitor may be present during the preparation of the nitrosamine precursor, e.g. amine oxides, to inhibit the formation of nitrosamines and nitrites during preparation, thereby producing a nitrosamine precursor having a low initial value for total NO. Further additions of the synergistic inhibitor may be made to the product if required after preparation.

The individual components of the synergistic inhibitor may also be added separately to the nitrosamine precursors. For example, the required amount of either may be present during the preparation of the nitrosamine precursor to inhibit the initial formation of nitrosamines and nitrites, with the remaining inhibitor added to the nitrosamine precursor after preparation.

In an preferred embodiment of the present invention, the complete synergistic inhibitor is present during preparation of an amine oxide, or alternatively (i) is present during preparation of an amine oxide and (ii) is added afterwards.

The complete synergistic inhibitor may be used in a sufficient proportion to effect inhibition e.g. in a quantity of from 0.01% to 5% by weight, based on the total weight of the product containing the nitrosamine precursor. Preferably 0.05% to 2.5% w/w of the synergistic inhibitor is used, more preferably 0.1% to 1.5% w/w, e.g. 0.15% to 1.0% w/w.

4

## An improved method for the preparation of amine oxides.

According to a particular embodiment of the present invention an improved method for the production of amine oxides is provided wherein a tertiary amine is reacted with hydrogen peroxide in the presence of a carbonate and/or bicarbonate catalyst and either a synergistic inhibitor according to the invention or component (i) thereof, to produce said amine oxide having extremely low levels of total NO. Where component (i) is used above, component (ii) is preferably added afterwards.

The tertiary amines that may be used in the improved process of our invention are typically amines of the general formula $R^1 R^2 R^3 N$, wherein $R^1$, $R^2$ and $R^3$ represent straight or branched chain alkyl groups, alkenyl groups or aralkyl groups which may be the same or different. They may be lower alkyl groups, i.e., of from 1 to 7, preferably 1 to 4, carbon atoms, but in a preferred embodiment of this invention, the tertiary amines may instead be represented by the general formula $(R)_m (R^1)_n N$, wherein $m = 1$ or 2 and $n = (3-m)$.

The R groups, which may be the same or different, represent in this case $C_8$-$C_{24}$ alkyl or alkenyl polyalkyleneoxy groups, $C_7$-$C_{23}$ esteralkyl or alkenyl groups, or amidoalkyl or alkenyl groups, and the $R^1$ groups, which may also be the same or different, represent $C_1$-$C_4$ alkyl, alkoxy or hydroxyalkyl, or polyalkyleneoxy groups.

The alkyleneoxy groups are preferably polyethyleneoxy groups or polypropyleneoxy or mixed ethyleneoxy propyleneoxy groups containing between 1 and 20 ethyleneoxy and/or propyleneoxy groups. The amidoalkyl groups are preferably $C_7$-$C_{23}$ alkyl or alkenyl-amidopropyl groups.

In a particularly preferred embodiment of the present invention the amine is an alkyl dimethyl amine e.g. lauryl/myristyl dimethyl amine.

The bicarbonate and/or carbonate may suitably comprise a bicarbonate such as sodium bicarbonate. Alternatively, other water soluble bicarbonates, e.g., alkali metal bicarbonates such as potassium bicarbonate or lithium bicarbonate, or alkaline earth metal bicarbonates such as magnesium or calcium bicarbonate may be present. Preferably, the catalyst may comprise a carbonate such as an alkali metal, alkaline earth metal or ammonium carbonate, e.g., sodium, magnesium or calcium carbonate, or a mixture of bicarbonate and carbonate.

The molar ratio of bicarbonate to carbonate preferably lies in the range from 0.01:1 to 1:0.01, especially 1:1, provided that the pH of the composition is not high enough to cause significant decomposition of the hydrogen peroxide during the preparation of amine oxides.

Preferably the reaction occurs in the presence of from 0.2 to 20% by weight based on the weight of amine of said catalyst and consisting of a bicarbonate and/or carbonate as described in GB 2 252 320. Preferably the amount of carbonate or bicarbonate is from 0.5 to 5% based on the initial weight of amine, most preferably 0.75 to 2% e.g. 0.8 to 1.5%.

Preferably the pH of the reaction mixture during the preparation of the amine oxide should be less than 10, e.g. less than 9.8, typically less than 9.5. However, when the synergistic inhibitor is added to the product after preparation, such a constraint does not arise and therefore higher pH values may be tolerated depending on the desired end use.

In a particular embodiment of the present invention the synergistic inhibitor comprises (i) sodium hydroxyethylamine bis (methylene phosphonate) and (ii) sodium diethylenetriamine pentakis (methylene phosphonate) in a weight ratio of from 5:1 to 1:2. The synergistic inhibitor is preferably present in said amine oxide in a total amount from 0.01% - 5% by weight based on the weight of the total amine composition, preferably 0.25% to 1.25% by weight.

The improved preparation of amines is typically carried out in aqueous solution. To avoid contamination by polyvalent anions that tend to cause (or accelerate) decomposition of the hydrogen peroxide, ultra high purity reagents are preferably used. The required concentration of product depends on the particular tertiary amine starting material employed, since those that are preferred for this embodiment of the present invention give rise to oxide products with surfactant properties which form a mobile $L_1$ phase at concentrations up to about 30% by weight based on total weight of reaction mixture, subject to exact chemical nature. At higher concentrations the amine oxide products tend to form an immobile M phase. Preferably, water is added to the reaction vessel in the form of aqueous hydrogen peroxide such that the final concentrations, e.g. 60 to 80% amine oxide (by weight based on total weight of reaction mixture) form a mobile G-phase. This option is, however, often impractical in commercial scale manufacture due to the narrow concentration range within which the G-phase is usually confined and consequently the most preferred embodiment is usually the most concentrated $L_1$ phase attainable, typically about 30% by weight based on total weight of reaction mixture. Higher concentrations may be achieved in the presence of phase modifiers such as solvents, cosurfactants, hydrotropes or electrolyte salts which may raise the $L_1$ to M phase boundary or broaden the concentration range of the G-phase. On a large scale, temperature rise may be a problem during the reaction, as conditions become closer to adiabatic, hence the use of a heel of amine oxide product is preferable since this limits the extent of the exotherm, by avoiding the induction period as the system passes from 2 phase to 1 phase.

In the method of the present invention, the amine and hydrogen peroxide are mixed together in a molar ratio of (amine: hydrogen peroxide) which may typically range from 1:0.9 through to 1:1.1. The most preferred ratio is 1:1, i.e., a stoichiometric amount. The presence of a substantial stoichiometric excess of hydrogen peroxide, e.g., more than 0.05% excess makes it difficult to obtain low levels of total NO and requires higher levels of stabiliser to achieve nitrosamine levels below 50ppb. Generally we prefer to operate between an (amine: hydrogen peroxide) of 1:1 to 1:1.09 w/w.

Optionally, a non phosphonate sequestrant such as EDTA may be used in the preparation to chelate the metal irons present in the composition which catalyse the decomposition of hydrogen peroxide. Alternative sequestrants include other chelating agents such as pyrophosphate. We prefer that such transition metal chelants or sequestrants should be present if the phosphonate is added after the commencement of the preparation. They accelerate the reaction but do not have any significant effect on the nitrosamine levels.

The inventors have found that, in the presence of bicarbonate and/or carbonate in the proportions specified above, and/or in the presence of phosphonate, temperature need not be such a crucial factor in limiting the formation of nitrosamine as by-products as is implied by the prior art. European Patent Application 88306270.3 states that in order to inhibit the formation of nitrosamines, the preparation of amine oxide must be conducted at 45ºC or lower; most preferably below 30ºC. This then necessitates the usage of a promoter to raise the slow reaction rate.

According to the method of the present invention the action of the synergistic inhibitor towards nitrosamine and nitrite formation, reduces the importance of minimising the temperature during the preparation, storage and/or heating of amine oxides. Accordingly, the temperature of the reaction vessel when carrying out the process of the present invention, with the catalyst present, may initially range from 2°C to 85°C, with the most preferred temperature range being 30°C to 50°C, with a typical operating temperature of approximately 40°C.

The reaction time for the preparation of the amine oxide depends upon the conditions employed typically being 0.1 - 24 hours, preferably 0.25 - 6 hours, more preferably 1-3.5 hours.

The improved method of preparation and analysis of said amine oxides is further illustrated with reference to Example 1.

The present invention will be further illustrated by the following Examples in which all percentages are by weight based on the total weight of the product or reaction mixture as appropriate, except where the context requires otherwise:-

## EXAMPLES

### Example 1 - Preparation of (tertiary) amine oxides in the presence of nitrosamine/nitrite inhibitors.

(i) In a flask, a sufficient quantity to produce a 30% w/w active amine oxide product of unstabilised (alkyl dimethyl) amine (e.g. EMPIGEN AB), inhibitor(s), catalyst (e.g. sodium carbonate) and water were mixed together, and the resulting reaction mixture stirred and warmed to 40°C using a water bath.

(ii) An initial charge of approximately 20% w/w of the total mass of hydrogen peroxide (35% w/w concentration) required to produce the amine oxide from the amine, was added to the flask, and the reaction mixture allowed to continue stirring for approximately a further 30 minutes until the reaction exotherm had been overcome.

(iii) The remaining charge of hydrogen peroxide was added to the reaction mixture over a further period of 30-45 minutes whilst stirring was maintained. When the total requisite amount of hydrogen peroxide had been added, the reaction was allowed to proceed until such time that the analysis of aliquots taken from the reaction mixture suggested the reaction was complete.

(iv) The reaction was deemed to have proceeded to completion and the amine oxide to have been formed when the analysis of an aliquot showed the reaction mixture to contain ≤ 0.3% w/w free amine, and ≤0.1% w/w hydrogen peroxide.

(v) If the analysis showed a high free amine content, but a low hydrogen peroxide content in the reaction mixture, a further small addition of hydrogen peroxide was made and the reaction allowed to proceed for a short time, for example 30 minutes, after which time a further aliquot was taken and analysed. This procedure was repeated until an aliquot taken from the reaction mixture showed levels of free amine and hydrogen peroxide content at or below those specified above, indicating that the reaction had reached completion and the amine had been fully converted to the amine oxide.

(vi) The amine oxide produced by the above method, was then analysed for the presence of nitrosamine and nitrite, by decomposition of the nitrosamine and nitrite with hydrogen bromide and acetic acid in an organic solvent to produce nitric oxide, with the analysis carried out by a chemiluminescence technique. Nitrosamine is determined e.g. from a separate aliquot after destruction of the nitrite with a sulphamic acid solution. The results are expressed as ppb NO.

'EMPIGEN' is a Registered Trade Mark of Albright & Wilson Limited.

**Example 2 - Storage stability (45°C) of an amine oxide in the absence of the synergistic nitrosamine/nitrite inhibitor.**

Two amine oxide samples were prepared in the absence of the synergistic inhibitor following the method of Example 1, with no additions of inhibitor made to the sample prior to storage at 45°C. Table 1 (below) shows the levels of nitrosamine and nitrite contamination in the amine oxide samples determined during storage for 8 weeks at 45°C. Samples were analysed for nitrosamine and nitrite content according to the method outlined in Example 1.

Both samples A and B were prepared using ultra high purity water and 1% w/w sodium bicarbonate catalyst. 0.1% w/w and 0.25% w/w EDTA was present in samples A and B respectively.

**Table 1 :- Nitrosamine and nitrite contamination in amine oxide samples in the absence of the synergistic inhibitor (stored at 45°C)**

| | nitrosamine ppb storage time (weeks) | | | | | nitrite (ppb) storage time (weeks) | | | | |
|---|---|---|---|---|---|---|---|---|---|---|
| | 0 | 2 | 4 | 6 | 8 | 0 | 2 | 4 | 6 | 8 |
| Sample A | 22 | 19 | 68 | 71 | 155 | 428 | 815 | 770 | 843 | 1210 |
| Sample B | 19 | 46 | - | 100 | 122 | 221 | 796 | - | 894 | 1521 |

All results are quoted as ppb NO.

Both samples A and B, in the absence of any inhibitors according to the present invention, exhibit rapid contamination at 45°C by nitrosamine and nitrite. The contamination from nitrite is particularly rapid.

**Example 3 :- Nitrosamine and nitrite contamination in amine oxide samples prepared in the presence of the synergistic inhibitor (stored at 45° C)**

Amine oxide samples were prepared according to the method given in Example 1 with the appropriate inhibitor added during stage (i) i.e. pre-addition, full particulars of the inhibitors added are given in Table 2 below. No further additions of inhibitor were made to the amine oxides prior to storage at 45° C. i.e. no post addition of inhibitor.

The levels of nitrosamine and nitrite contamination were determined according to the method outlined in Example 1 during the storage period with the results given below in Table 2:

Ultra high purity water and 1% w/w sodium bicarbonate catalyst were used during the preparation.

## Table 2 :- Nitrosamine and nitrite contamination in amine oxide samples containing synergistic inhibitors (stored at 45°C)

| Sample | Inhibitor (% w/w) | ppb nitrosamine storage time (weeks) | | | | | ppb nitrite storage time (weeks) | | | | |
|---|---|---|---|---|---|---|---|---|---|---|---|
| | | 0 | 2 | 4 | 6 | 8 | 0 | 2 | 4 | 6 | 8 |
| 1 | 0.25% - inhibitor 1 | 7 | 83 | 213* | 214* | 114* | 134 | 330 | 953 | 1029 | 147 |
| 2 | 0.25% - inhibitor 2 | 7 | 13 | 20 | 23 | 36 | 183 | 270 | 483 | 665 | 75 |
| 3 | 0.125% - inhibitor 1 + 0.125% - inhibitor 2 | 9 | 11 | 16 | 10 | 21 | 185 | 249 | 334 | 356 | 35 |

\*     the accuracy of these results cannot be guaranteed due to a degree of experimental error. However, the general trend may be observed.

Inhibitor 1 = sodium salt of hydroxyethylamine bis methylene phosphonic acid (Briquest 221)

Inhibitor 2 = pentakis diethylenetriamine methylene phosphonate (Briquest 543)

'BRIQUEST' is a Registered Trade Mark of Albright & Wilson Limited

A comparison of the results for Samples 1, 2 and 3 above, with those obtained from Samples A and B in Example 2, show the reduction in nitrosamine and nitrite contamination resulting from the presence of an inhibitor.

Sample 3 clearly shows a significant improvement in the inhibition of both nitrosamine and nitrite contamination with respect to Samples 1 and 2, both of which contain only a single inhibitor at the same total concentration Inhibitor 1 is an effective inhibitor for the preparation of nitrosamine precursors, especially with respect to contamination by nitrite. However its inhibitory effect upon storage is inferior with respect to Inhibitor 2.

If the inhibition of nitrosamine and nitrite in Sample 3 was due solely to the separate inhibitory actions of the individual inhibitors samples containing a 50/50 mix of the Inhibitors would not be expected to show lower nitrosamine and nitrite levels to those of single inhibitor type samples, all samples containing an equal total amount of inhibitor. This is however the result shown by Sample 3.

Thus the use of Inhibitor 1 or 2 alone does not reduce the level of nitrosamine and nitrite in amine oxides to that achievable by the use of the synergistic inhibitor, according to the present invention, where the total level of inhibitor is equal in each cases. The novel synergistic inhibitor of sample 3 is extremely effective in reducing the levels of both nitrosamines and nitrites especially with regard to the inhibition of nitrite formation.

**Example 4:- Post addition of Inhibitor 2 to compositions of Sample 1, (storage at 45°C)**

To portions of Sample 1 (containing 0.25% Inhibitor 1) additions of Inhibitor 2, as given below in Table 3, were added immediately after preparation of the amine oxide. The samples thus produced were stored at 45°C. Results for the analysis of nitrosamine and nitrite content of the samples during the storage period are given below in Table 3. The analysis of the contaminants is in accordance with Example 1.

Ultra high purity water and 1% w/w sodium bicarbonate catalyst were used during the preparation.

## Table 3 :- Post addition of Inhibitor 2

| Sample | Inhibitor 2 (%w/w)(post addition) | ppb nitrosamine storage time (weeks) | | | | | ppb nitrite storage time (weeks) | | | | |
|---|---|---|---|---|---|---|---|---|---|---|---|
| | | 0 | 2 | 4 | 6 | 8 | 0 | 2 | 4 | 6 | 8 |
| 1 | - | 7 | 83 | 213* | 214* | 114* | 134 | 330 | 953 | 1029 | - |
| 4 | 0.1 % - 3 | 7 | 5 | 10 | 9 | 7 | 134 | 157 | 170 | 274 | 326 |
| 5 | 0.25 % - 3 | 7 | 6 | 6 | 8 | 10 | 134 | 170 | 188 | 236 | 290 |
| 6 | 0.5 % - 3 | 7 | 9 | 7 | 11 | 6 | 134 | 148 | 167 | 245 | 269 |

\* See Example 3.

Thus it can be seen from samples 4, 5 and 6, that increasing the level of Inhibitor 2 between 0.1% and 0.5% (w/w),in the presence of Inhibitor 1 has little effect in improving the inhibition of nitrosamine and nitrite formation in amine oxides. This further illustrates the synergistic action of the inhibitor mixture of the present invention.

**Example 5 :- The effect of the Inhibitor 1 in Example 4**

An amine oxide (Sample 8) was prepared according to the method of Example 1, in the presence of Inhibitor 2 only (0.5% w/w), i.e. same total inhibitor content to Sample 5.

Table 4 below shows a comparison between Sample 5 and Sample 8. Sample 5 was stored at 45°C, and Sample 8 at 40°C.

Ultra High purity water and 1% w/w sodium bicarbonate catalyst were used in the preparation of Sample 5, with 1% of the catalyst and mains water used for the preparation Sample 8.

Table 4

| A comparison of Sample 5 and Sample 8 | | | | |
|---|---|---|---|---|
| Sample | ppb nitrosamine storage time (weeks) | | ppb nitrite storage time (weeks) | |
| | 0 | 2 | 0 | 2 |
| 5 | 7 | 6 | 134 | 170 |
| 8 | 9 | 16 | 185 | 334 |

The results in Table 4 show the reduction in nitrosamine and nitrite contamination in the amine oxides of Example 4 is not due solely to the effect of Inhibitor 2, as the presence of Inhibitor 1 can be seen to have an effect in Sample 5. Sample 5 clearly demonstrates the synergism between Inhibitor 1 and Inhibitor 2. The storage conditions for Sample 8 are not as harsh as those for Sample 5, the former being 5°C lower. Under the higher storage temperature of 45°C the contamination levels for Sample 8 would be expected to increase and thus the effect of the synergism would be further illustrated.

**Example 6 :- Addition of Inhibitor 2 (a) during step (i) of the preparation and (b) prior to storage**

Sample 7 in Table 5 (below) was prepared according to the method of Example 1, with the synergistic Inhibitors 1 and 2 added in stage (i) of the preparation. i.e. pre addition of Inhibitor 2. Sample 5 in Table 5 was prepared according to the method of Example 1, with Inhibitor 1 added during stage (i) of the preparation and Inhibitor 2 added after the preparation of the amine oxide was complete, i.e. post addition of Inhibitor 2. Both samples contained 0.25% w/w of Inhibitor 1 and 0.25% w/w Inhibitor 2 i.e. 0.5% w/w total synergistic inhibitor.

Samples 5 and 7 were stored at 45°C for a period of 8 weeks. The nitrosamine and nitrite content of both samples was determined by the method earlier described in Example 1.

Ultra high purity water and 1% w/w sodium bicarbonate catalyst was used in the preparation.

## Table 5 :- A comparison of pre- and post- addition of Inhibitor 2.

| Sample and addition of Inhibitor 2 | nitrosamine (ppb) storage time (weeks) | | | | | nitrite (ppb) storage time (weeks) | | | | |
|---|---|---|---|---|---|---|---|---|---|---|
| | 0 | 2 | 4 | 6 | 8 | 0 | 2 | 4 | 6 | 8 |
| 7-Pre | 6 | 9 | 10 | - | 10 | 120 | 165 | 189 | - | 165 |
| 5-Post | 7 | 6 | 6 | 8 | 10 | 134 | 170 | 188 | 236 | 290 |

Samples 5 and 7 exhibit extremely similar results for the levels of nitrosamine and nitrite contamination in said samples over the eight week storage period at 45°C.

The synergistic inhibitor mixture comprising Inhibitor 1 and 2 may therefore be added to the reaction mixture and the amine oxide prepared in the presence of the synergistic inhibitor mixture, or alternatively Inhibitor 1 may be added prior to preparation of the amine oxide i.e. in step (i) of Example 1, with the second component of said synergistic mixture, here Inhibitor 2, added immediately upon the formation of the amine oxide and prior to storage. Both pre- and post- addition of the second component of the synergistic inhibiter is shown to be effective in reducing nitrosamine and nitrite contamination in nitrosamine precursors.

### Example 7 :- The minimum quantity of Inhibitor 2 required to exhibit synergism with Inhibitor 1

An amine oxide was prepared according to the method of Example 1 with 0.25% w/w Inhibitor 1 added during stage (i) of the preparation.

Additions of Inhibitor 2 were made to portions of said amine oxide immediately prior to storage at 45°C i.e. post-addition of Inhibitor 2 as detailed below. Table 6 displays the analysis results for nitrosamine and nitrite contamination of the amine oxide samples during a four week storage period at 45°C.

The nitrosamine and nitrite levels of the samples in Table 6 were determined according to the analytical technique of Example 1.

Ultra high purity water and 1% w/w sodium bicarbonate catalyst were used in the preparation.

Table 6

| Determination of the minimum quantity of Inhibitor 2 required to effect synergism with Inhibitor 1 | | | | | | | |
|---|---|---|---|---|---|---|---|
| Sample | Inhibitor 2 post addition (% w/w) | nitrosamine (ppb) storage time weeks) | | | nitrite (ppb) storage time (weeks) | | |
| | | 0 | 2 | 4 | 0 | 2 | 4 |
| 9 | - | 17 | 111 | 146 | 211 | 329 | 660 |
| 10 | 0.025% | 17 | 16 | 24 | 211 | 256 | 435 |
| 11 | 0.05 % | 17 | 15 | 14 | 211 | 236 | 322 |
| 12 | 0.225% | 17 | 15 | 17 | 211 | 224 | 303 |

Samples 11 and 12 show very similar analysis results for both nitrosamine and nitrite content suggesting the synergism of the inhibitory mixture to be virtually independent of the concentration of Inhibitor 2 over a 0.05% w/w - 0.225% w/w concentration range. This is in agreement with the results of Example 4. The synergistic action of the inhibitor is exhibited down to levels of 0.025% w/w Inhibitor 2 in the presence of 0.25% w/w of Inhibitor 1 (i.e. 10:1 w/w mixture of Inhibitor 1 to Inhibitor 2). The synergistic effect at the 10:1 (Inhibitor 1 to Inhibitor 2) ratio is slightly less marked than the inhibitory effect at the higher ratios of Examples 11 and 12, i.e. (4.5:1 and 1.1:1.)

## Example 8:- Alternative synergistic inhibitors

An amine oxide was prepared and analysed according to the method of Example 1. All samples used 1% sodium bicarbonate catalyst and ultra high purity water. Samples were stored at 45°c for 4 weeks. Table 7 gives the quantity and method of addition of each inhibitor.

Table 7

| Nitrosamine and nitrite contamination in amine oxide samples containing synergistic inhibitors (stored at 45°c) | | | | | | | |
|---|---|---|---|---|---|---|---|
| Sample | Inhibitor | nitrosamine (ppb) storage time weeks) | | | nitrite (ppb) storage time (weeks) | | |
| | | 0 | 2 | 4 | 0 | 2 | 4 |
| 9 | 0.25% - 1 pre addition | 17 | 111 | 146 | 211 | 329 | 660 |
| 13 | 0.25% w/w - 3 pre addition | 17 | 16 | 16 | 375 | 418 | 544 |
| 14 | 0.25% w/w -1 + 0.25% - 3 post addition | 5 | 5 | 5 | 108 | 168 | 183 |
| Inhibitor 3 = sodium tetraethylenepentamine heptakis-(methylenephosphonate) (Briquest 785) | | | | | | | |

Although the samples do not contain the same total level of inhibitor the effect of synergism of the inhibitor mix can be observed. In example 7 the effect of synergism is shown to be independent of the concentration of the second component.

## Claims

1. A synergistic nitrosamine and/or nitrite inhibitor comprising :-
   (i) a compound of formula:-

   $R_y N(O)_x (CH_2 PO_3 M_2)_{(3-y)}$

   where y is 1 or 2, x is 1 or 0,R is an alkyl group having up to 6 carbon atoms or a hydroxyalkyl, carboxyalkyl or polyoxethylene group having 2 to 6 carbon atoms and M is hydrogen, or a cation such that the compound is water soluble; and
   (ii) a synergistic proportion of a compound of general formula :-

   $(M_2 O_3 PCH_2)_2 N(O)_x [(CH_2)_m N(O)_x CH_2 PO_3 M_2]_n CH_2 PO_3 M_2$

   wherein n is independently from 0 to 8, m is 2 or 3, and x and M are as hereinbefore defined.

2. A synergistic inhibitor comprising :-
   (i) an alkanolamine bis (methylene phosphonic) acid, or its N-oxide, or a water soluble salt thereof; and
   (ii) a synergistic proportion of a polyalkyleneamine poly(methylene phosphonic) acid, or its N-oxide, or a water soluble salt thereof.

3. A synergistic inhibitor according to Claim 1 wherein R is $HOCH_2 CH_2$ and y is 1.

4. A synergistic inhibitor according to either of Claims 1 and 3 wherein n is from 1 to 4 and m is 2.

5. A synergistic inhibitor according to Claim 4 wherein n is 2 or 3.

6. A synergistic inhibitor according to any preceding claim, wherein the weight ratio of (i):(ii) in said synergistic inhibitor lies within the range 15:1 to 1:15.

7. A synergistic inhibitor according to Claim 6, wherein said weight ratio of (i):(ii) lies within the range 5:1 to 1:5.

8. A synergistic inhibitor according to Claim 7, wherein said weight ratio of (i):(ii) is at least one.

9. A synergistic inhibitor according to any preceding claim which additionally comprises a bicarbonate and/or carbonate.

10. A synergistic inhibitor according to Claim 9 wherein the molar ratio of said bicarbonate to carbonate lies in the range from 0.01:1 to 1:0.01.

11. A synergistic inhibitor according to Claim 10, wherein said bicarbonate and/or carbonate is present in a proportion of from 5% to 95% by weight based on the total weight of said inhibitor.

12. A synergistic inhibitor according to Claim 11, wherein said bicarbonate and/or carbonate is an alkali metal salt.

13. A synergistic inhibitor according to Claim 12, wherein said bicarbonate and/or carbonate is sodium bicarbonate and/or sodium carbonate.

14. A synergistic inhibitor according to any preceding claim wherein (i) is an alkali metal salt of an N-oxide.

15. A synergistic inhibitor according to any preceding claim wherein (ii) is an alkali metal salt of an N-oxide.

16. A method of inhibiting the conversion of potential nitrosamine precursors to nitrosamine and/or nitrite during the preparation, storage and/or heating of said precursors, which method comprises adding thereto a proportion of a synergistic inhibitor according to any preceding claim effective to inhibit said conversion.

17. A method according to Claim 16, wherein said nitrosamine precursor is an amine, amine oxide or alkanolamide.

18. A method according to Claim 17, wherein said amine precursor is a secondary or tertiary amine or amine oxide.

19. A method for the preparation of amine oxides, said method comprising reacting an amine with hydrogen peroxide in the presence of a sufficient amount of hydroxyethylamine bis (methylene phosphonic) acid N-oxide, or a water soluble salt thereof, to inhibit the formulation of nitrosamine.

20. A method for the preparation of amine oxides comprising reacting an amine with hydrogen peroxide in a reaction mixture containing at least the component (i) of said synergistic inhibitor according to any one of Claims 1 to 15 and adding component (ii) of said synergistic inhibitor to said reaction mixture before, during or after said reaction.

21. A method for the preparation of amine oxides according to either of Claims 19 and 20, wherein the reaction of said amine with hydrogen peroxide occurs in the presence of a bicarbonate and/or carbonate.

22. A method for the preparation of amine oxides according to any of Claims 19 to 21 wherein said amine is of the general formula:-

$R^1R^2R^3N$, wherein $R^1$, $R^2$ and $R^3$ which may be the same or different represent straight or branched chain alkyl groups, alkenyl groups or aralkyl groups having in each case from 1 to 7 carbon atoms; or $(R)_m(R^1)_nN$ wherein m is 1 or 2, n is (3-m), the R groups, which may be the same or different, represent $C_8$-$C_{24}$ alkyl or alkenyl polyalkyleneoxy groups, $C_7$-$C_{23}$ esteralkyl or alkenyl groups, or amidoalkyl or alkenyl groups, and the $R^1$ groups, which may also be the same or different, represent

EP 0 608 873 A1

$C_1$-$C_4$ alkyl, alkoxy or hydroxyalkyl, or polyalkyleneoxy groups.

23. A method for the preparation of amine oxides according to Claim 22, wherein said amine is of the general formula $R_{(m)}R^1_{(n)}N$ wherein m is 1 and n is 2.

24. A method for the preparation of amine oxides according to Claim 23, wherein said amine is an alkyl dimethylamine.

25. A method for the preparation of amine oxides according to any one of Claims 20 to 24, wherein (ii) is added to said amine oxide after preparation thereof.

26. A method for the preparation of amine oxides according to any of Claims 20 to 25 wherein said synergistic inhibitor is present in a total proportion of from 0.01% to 5% by weight based on the initial weight of said amine used in said preparation.

27. A method for the preparation of amine oxides according to Claim 26, wherein said synergistic inhibitor is present in a proportion of from 0.25% to 1.25% by weight based on the initial weight of amine used in said preparation.

28. A method for the preparation of amine oxides according to any preceding claim wherein the final concentration of the amine oxide is up to 80% by weight based on the total weight of the mixture formed.

29. A method for the preparation of amine oxides according to Claim 28, wherein said amine oxide is present as a "G" or "L1" phase.

30. A method for the preparation of amine oxides according to any of Claims 19 to 29, wherein the molar ratio of amine : hydrogen peroxide is 1.09:1 to 1:1.1.

31. A method for the preparation of amine oxides according to any of Claims 19 to 30, wherein the amine oxide is prepared in the presence of ethylene diamine tetracetic acid or its water soluble salts.

32. A method for the inhibition of the conversion of potential nitrosamine precursors to nitrosamine wherein said precursors are treated to produce low initial levels of nitrosamines and nitrites, said method comprising the addition of a synergistic inhibitor according to any one of Claims 1 to 15 to said precursors following said treatment.

33. A method according to Claim 32 wherein said treatment comprises irradiation with ultra violet rays.

34. A composition comprising a nitrosamine precursor and a synergistic inhibitor according to any one of Claims 1 to 15 in a proportion effective to inhibit the formation of nitrosamines.

35. A composition according to Claim 34 comprising an amine oxide.

36. A composition according to either of Claims 34 and 35, wherein said composition comprises from 0.01% to 5% by weight of said synergistic inhibitor, based on the total weight of said composition.

37. A composition according to Claim 36, wherein said composition comprises from 0.1% to 1.5% by weight of said synergistic inhibitor, based on the total weight of said composition.

38. A composition according to any of Claims 34 to 37 wherein the total proportion of (i) and (ii) in said composition is between 0.01% to 5% by weight based on the total weight of the product.

39. A composition according to Claim 38 wherein the total proportion of (i) and (ii) in said composition is between 0.1% and 1.5% by weight based on the total weight of the product.

40. A composition according to any of Claims 34 to 39 wherein said composition comprises a total of 0.5% to 5% by weight of an alkali metal bicarbonate and/or carbonate, based on the initial weight of amine.

13

**41.** A composition according to Claim 40, wherein said composition comprises a total of 0.75% to 2% by weight of sodium bicarbonate and/or sodium carbonate, based on the initial weight of amine.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,Y | EP-A-0 409 043 (INTEROX AMERICA)<br>* the whole document *<br>--- | 1-41 | C07C291/04<br>C09K15/32 |
| D,Y | EP-A-0 498 346 (ALBRIGHT & WILSON LTD.)<br>* the whole document *<br>--- | 1-41 | |
| D,P,<br>Y | EP-A-0 553 800 (ALBRIGHT & WILSON LTD.)<br><br>* the whole document *<br>----- | 1-41 | |

TECHNICAL FIELDS
SEARCHED      (Int.Cl.5)

C07C
C09K

The present search report has been drawn up for all claims

| Place of search | Date of completion of the search | Examiner |
|---|---|---|
| THE HAGUE | 23 March 1994 | Beslier, L |